# EUROPEAN PATENT SPECIFICATION

(11) **EP 2 094 650 B1**
(45) Date of publication and mention of the grant of the patent: **21.09.2011**
(21) Application number: 07847910.2
(22) Date of filing: 06.12.2007
(51) Int. Cl.: C07C 255/31, C07C 43/303, C07C 47/11, C07C 47/225, C07C 49/21, C11B 9/00

(54) **USE OF CAMPHOLENIC DERIVATIVES AS FRAGRANT INGREDIENTS IN PERFUMERY AND FLAVOURING**
VERWENDUNG VON CAMPHOLENDERIVATEN ALS DUFTSTOFFBESTANDTEILE IN PARFÜMEN UND GESCHMACKSTOFFEN
UTILISATION DE DÉRIVÉS CAMPHOLÉNIQUES COMME INGRÉDIENTS PARFUMANTS EN PARFUMERIE ET EN AROMATISATION

(30) Priority: 08.12.2006 EP 06291891; 08.12.2006 US 873586 P
(43) Date of publication of application: 02.09.2009
(73) Proprietor: V. Mane Fils, 06620 Bar sur Loup (FR)
(72) Inventor: MANE, Jean, 06130 Grasse (FR); PLESSIS, Caroline, 06620 Bar sur Loup (FR); CHANOT, Jean-Jacques, 06530 Speracedes (FR)
(74) Representative: Cabinet Plasseraud
(86) International application number: PCT/EP2007/063435
(87) International publication number: WO 2008/068310

(56) References cited:
- EP-A1- 1 067 118
- EP-A2- 0 466 019
- DATABASE CAPLUS [Online] CHEMICAL ABSTRACTS SERVICE, COLUMBUS, OHIO, US; DERDZINSKI, KRZYSZTOF ET AL: "Preparation of 3-methyl-6-(2,2,3-trimethyl-3-cyclopenten- 1-yl)-2-hexenol stereoisomers as perfume ingredients" XP002436398 retrieved from STN Database accession no. 1988:529379 & PL 130 019 B2 (POLITECHNIKA WROCLAWSKA, POL.) 30 June 1984 (1984-06-30)
- WAWRZENCZYK, C. ET AL: "Juvenoids with cyclopentene ring. Synthesis of isopropyl 3,7-dimethyl-10-(2,2,3-trimethylcyclopent- 3-en-1-yl)deca-2,8-dienoate and deca-2,4,8-trienoate" JOURNAL FUER PRAKTISCHE CHEMIE, vol. 326, no. 2, 1984, pages 213-221, XP009084701

## Description

### [FIELD OF THE INVENTION]

The present invention relates to the field of fragrances and flavours. More particularly, the invention relates to new campholenic derivatives and to their use in the fields of perfumery and flavouring.

### [BACKGROUND]

Several campholenic derivatives, having a C₁-C₄ or C₁-C₅ alkyl or C₂-C₄ or C₂-C₅ alkenyl chain branched in position 1' of the campholene cycle, have been described in the literature as odorants, as in EP 0 203 528, EP 0 466 019, EP 1 008 579, EP 0 841 318 or in US 5,057,158. Particularly, these documents deal with cyclopentanebutanol, cyclopentenbutenol and cyclopentenepentenol derivatives, presenting a characteristic sandalwood oil odour.

### [PROBLEM TO BE SOLVED]

The Applicant focused on a new family of cyclopentane or cyclopentene compounds substituted with a side chain of at least 6 carbon atoms. The Applicant found that one compound of this family, 3-methyl-6-(2,2,3-trimethyl-cyclopent-3-enyl)-hex-4-enal was known but no organoleptic properties were ever disclosed for this compound.

Surprisingly, and despite the fact that campholenic derivatives are usually known for their sandalwood fragrance, the new campholenic derivatives presented unexpected odours, such as floral, fruity or marine.

The need of new compounds is of great importance for the development of the flavour and fragrance industry, which recently had to face stricter international regulatory requirements about the use of certain materials, as well as environmental concerns and customer demands for improved performance. Being able of manufacturing new fragrant compounds is therefore a challenge.

In other words, this new approach figures out a technical problem which is to propose new flavour or fragrant compounds, preferably by a simple and inexpensive manufacturing process.

### [DEFINITIONS]

The terms "fragrance" and "fragrant", as used herein, are used interchangeably whenever a compound or a mixture of compounds is referred to, which is intended to pleasantly stimulate the sense of smell.

The terms "flavour" and "flavouring", as used herein, are used interchangeably whenever a compound or a mixture of compounds is referred to, which is intended to stimulate the sense of taste and smell. Also in the meaning of the invention, the term "flavouring" relates to the flavouring of any liquid or solid, human or animal, in particular of drinks, dairy products, ice creams, soups, sauces, dips, dishes, meat products, culinary assistances, salted biscuits or snacks. It also means the flavouring of beers, wines and tobaccos.

The term "organoleptic compound", such as for example fragrances and flavours, as used herein, refers to compounds of the invention which stimulate the sense of smell or taste, and are thus perceived as having a characteristic odour and/or flavour.

The term "olfactory effective amount", as used herein, means a level or amount of fragrant/flavouring compound present in a material at which the incorporated compound exhibits a sensory effect.

By the term "masking" is meant reducing or eliminating malodour or bad flavour perception generated by one or more molecules entering in the composition of a product.

The term "alkyl" or "alkyl group", in the present invention, means any linear or branched saturated hydrocarbon chain, having preferably 1, 2, 3, 4 or 5 carbon atoms and herein referred to as C₁₋₅ alkyl group, such as for example methyl, ethyl, propyl, isopropyl, butyl, t-butyl, pentyl.

The term "alkenyl" or "alkenyl group", in the present invention, means any linear or branched mono or poly unsaturated hydrocarbon chain, having preferably 2, 3, 4 or 5 carbon atoms and herein referred to as C₂₋₅ alkenyl group, such as for example ethenyl, propenyl, butenyl, or pentenyl.

The term "isomer", in the present invention, means molecules having the same chemical formula, which means same number and types of atoms, but in which the atoms are arranged differently. The term "isomer" includes structural isomers, geometric isomers, optical isomers and stereoisomers.

This invention thus relates to campholenic derivative of general formula (I) 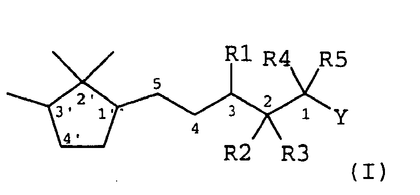 wherein
- R1, R2, R3, R4 and R5 represent, each independently, a hydrogen atom or a linear or branched C₁-C₅ alkyl or C₂-C₅ alkenyl group,
- Y represents a CN, a C(O)R6 or a CR6(ORα)(ORβ) group, wherein R6 represents a hydrogen atom or a linear or branched C₁-C₅ alkyl or C₂-C₅ alkenyl group,
   and Rα and Rβ represent simultaneously a linear or branched C₁-C₅ alkyl or C₂-C₅ alkenyl group or form together a cycle, and preferably Y represents CN, CHO, C(O)CH₃, C(O)C₂H₅, or CR6(ORα) (ORβ), wherein R6 represents a hydrogen atom, a methyl or an ethyl group and Rα and Rβ represent simultaneously a linear or branched C₁-C₅ alkyl or C₅-C₅ alkenyl group or form together a cycle
- the 5-membered ring is saturated or has a double bond between C3' and C4' in formula (I), and
- the side chain, optionally, has a double bond between C1 and C2 and/or between C3 and C4
- Provided that the derivative is not 3-methyl-6-(2,2,3-trimethyl-cyclopent-3-enyl)-hex-4-enal.

Advantageously, the compounds of the invention are those of general formula (I) hereabove, wherein
- R1, R2, R3, R4 and R5 represent, each independently, a hydrogen atom or a methyl or an ethyl group,
- Y represents a CN, a C(O)R6 or a CR6(ORα)(ORβ) group, wherein R6 represents a hydrogen atom or a linear or branched C₁-C₅ alkyl or C₂-C₅ alkenyl group,
   and Rα and Rβ represent simultaneously a linear or branched C₁-C₅ alkyl or C₂-C₅ alkenyl group or form together a cycle, and preferably Y represents CN, CHO, C(O)CH₃, C(O)C₂H₅, or CR6(ORα) (ORβ), wherein R6 represents a hydrogen atom, a methyl or an ethyl group and Rα and Rβ represent simultaneously a linear or branched C₁-C₅ alkyl or C₂-C₅ alkenyl group or form together a cycle,
- the 5-membered ring is saturated or has a double bond between C3' and C4' in formula (I), and
- the side chain, optionally, has a double bond between C1 and C2 and/or between C3 and C4
- Provided that the derivative is not 3-methyl-6-(2,2,3-trimethyl-cyclopent-3-enyl)-hex-4-enal.

Advantageously, the compounds of the invention are those of general formula (I) hereabove, wherein
- R1, R2, R3, R4 and R5 represent, each independently, a hydrogen atom or a linear or branched C₁-C₅ alkyl or C₂-C₅ alkenyl group,
   Y represents CN, CHO, C(O)CH₃, C(O)C₂H₅, or CR6(ORα)(ORβ), wherein R6 represents a hydrogen atom, a methyl or an ethyl group and Rα and Rβ represent simultaneously a linear or branched C₁-C₅ alkyl or C₂-C₅ alkenyl group or form together a cycle,
- the 5-membered ring is saturated or has a double bond between C3' and C4' in formula (I), and
- the side chain, optionally, has a double bond between C1 and C2 and/or between C3 and C4
- Provided that the derivative is not 3-methyl-6-(2,2,3-trimethyl-cyclopent-3-enyl)-hex-4-enal.

According to another advantageous embodiment, the compounds of the invention are those of general formula (I) hereabove, wherein
- R1, R2, R3, R4 and R5 represent, each independently, a hydrogen atom or a methyl or an ethyl group, and
- Y represents CN, CHO, C(O)CH₃, C(O)C₂H₅, or CR6(ORα)(ORβ), wherein R6 represents a hydrogen atom, a methyl or an ethyl group and Rα and Rβ represent simultaneously a linear or branched C₁-C₅ alkyl or C₂-C₅ alkenyl group or form together a cycle,
- the 5-membered ring and the side chain are unsaturated, preferably the side chain is mono-unsaturated
- Provided that the derivative is not 3-methyl-6-(2,2,3-trimethyl-cyclopent-3-enyl)-hex-4-enal.

According to another advantageous embodiment, the compounds of the invention are those of general formula (I) hereabove, wherein
- R1, R2, R3, R4 and R5 represent, each independently, a hydrogen atom or a linear or branched C₁-C₅ alkyl or C₂-C₅ alkenyl group,
- Y represents CN, CHO, C(O)CH₃, C(O)C₂H₅, or CR6(ORα)(ORβ), wherein R6 represents a hydrogen atom, a methyl or an ethyl group and Rα and Rβ represent simultaneously a linear or branched C₁-C₅ alkyl or C₂-C₅ alkenyl group or form together a cycle,
- the 5-membered ring and the side chain are unsaturated, preferably the side chain is mono-unsaturated
- Provided that the derivative is not 3-methyl-6-(2,2,3-trimethyl-cyclopent-3-enyl)-hex-4-enal.

According to a first embodiment of the invention, the campholenic derivatives of the invention are of general formula (Ia), 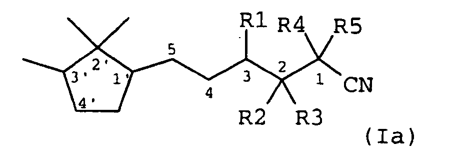 Wherein
- R1, R2, R3, R4 and R5 represent, each independently, a hydrogen atom or a linear or branched C₁-C₅ alkyl or C₂-C₅ alkenyl group, and preferably R1, R2, R3, R4 and R5 represent, each independently, a hydrogen atom or a methyl or an ethyl group,
- the 5-membered ring is saturated or has a double bond between C3' and C4', and
- the side chain is optionally unsaturated between C1 and C2 and/or between C3 and C4 in formula (Ia).

According to a second embodiment of the invention, the campholenic derivatives of the invention are of general formula (Ib), 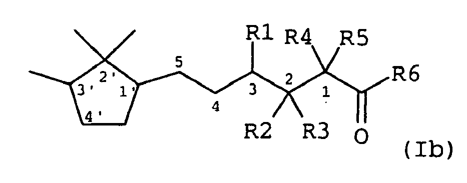 wherein
- R1, R2, R3, R4 and R5 represent, each independently, a hydrogen atom or a linear or branched C₁-C₅ alkyl or C₂-C₅ alkenyl group, and preferably R1, R2, R3, R4 and R5 represent, each independently, a hydrogen atom or a methyl or an ethyl group,
- the 5-membered ring is saturated or has a double bond between C3' and C4', and
- the side chain is optionally unsaturated between C1 and C2 and/or between C3 and C4 in formula (Ib), and
- R₆ represents a hydrogen atom, a linear or branched C₁₋₅ alkyl or C₂₋₅ alkenyl group, preferably, R₆ is a hydrogen atom or a methyl group, more preferably R₆ is a hydrogen atom,
- Provided that the derivative is not 3-methyl-6-(2,2,3-trimethyl-cyclopent-3-enyl)-hex-4-enal.

According to a third embodiment of the invention, the campholenic derivatives of the invention are of general formula (Ic), 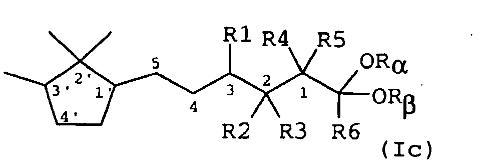 wherein
- R1, R2, R3, R4 and R5 represent, each independently, a hydrogen atom or a linear or branched C₁-C₅ alkyl or C₂-C₅ alkenyl group, and preferably R1, R2, R3, R4 and R5 represent, each independently, a hydrogen atom or a methyl or an ethyl group,
- the 5-membered ring is saturated or has a double bond between C3' and C4' in formula (Ic), and
- the side chain is optionally unsaturated between C1 and C2 and/or between C3 and C4 in formula (Ic), and
- R_{α} and R_{β} represent simultaneously a linear or branched C₁-C₅ alkyl or C₂-C₅ alkenyl group, preferably, R_{α} and R_{β} are simultaneously a methyl or an ethyl group, or
- R_{α} and R_{β} form together a cycle, preferably a 5-membered ring or a 6-membered ring.

This invention relates to the compounds of formula (I), as described above, but also to any of their various isomers.

According to a preferred embodiment of the invention, the campholenic derivatives of the invention are selected in the group comprising:
6-(2,2,3-trimethyl-cyclopent-3-enyl)-hex-4-enenitrile, (*4E*)-6-(2,2,3-trimethyl-cyclopent-3-enyl)-hex-4-enenitrile, (*4Z*)-6-(2,2,3-trimethyl-cyclopent-3-enyl)-hex-4-enenitrile, (+)-6-((1R)-2,2,3-trimethyl-cyclopent-3-enyl)-hex-4-enenitrile, (-)-6-((1*S*)-2,2,3-trimethyl-cyclopent-3-enyl)-hex-4-enenitrile, (*4E*)-6-((1R)-2,2,3-trimethyl-cyclopent-3-enyl)-hex-4-enenitrile, (*4Z*)-6-((1R)-2,2,3-trimethyl-cyclopent-3-enyl)-hex-4-enenitrile, (*4E*)-6-((1*S*)-2,2,3-trimethyl-cyclopent-3-enyl)-hex-4-enenitrile, (*4Z*)-6-((1S)-2,2,3-trimethyl-cyclopent-3-enyl)-hex-4-enenitrile, 6-(2,2,3-trimethyl-cyclopentyl)-hex-4-enenitrile, (*4E*)-6-(2,2,3-trimethyl-cyclopentyl)-hex-4-enenitrile, (*4Z)*-6-(2,2,3-trimethyl-cyclopentyl)-hex-4-enenitrile, 4-methyl-6-(2,2,3-trimethyl-cyclopent-3-enyl)-hex-4-enenitrile, (*4Z*)-4-methyl-6-(2,2,3-trimethyl-cyclopent-3-enyl)-hex-4-enenitrile, (*4E*)-4-methyl-6-(2,2,3-trimethyl-cyclopent-3-enyl)-hex-4-enenitrile, 4-methyl-6-(2,2,3-trimethyl-cyclopentyl)-hexanenitrile, 4-ethyl-6-(2,2,3-trimethyl-cyclopentyl)-hexanenitrile, 4-methyl-6-(2,2,3-trimethyl-cyclopentyl)-hex-3-enenitrile, 6-(2,2,3-trimethyl-cyclopent-3-enyl)-hex-4-enal, (*4E*)-6-(2,2,3-trimethyl-cyclopent-3-enyl)-hex-4-enal, (*4Z*)-6-(2,2,3-trimethyl-cyclopent-3-enyl)-hex-4-enal, (+)-6-((1R)-2,2,3-trimethyl-cyclopent-3-enyl)-hex-4-enal, (-)-6-((1*S*)-2,2,3-trimethyl-cyclopent-3-enyl)-hex-4-enal, (*4Z*)-6-((1R)-2,2,3-trimethyl-cyclopent-3-enyl)-hex-4-enal, (*4Z*)-6-((1S)-2,2,3-trimethyl-cyclopent-3-enyl)-hex-4-enal, (*4Z*)-6-((1S)-2,2,3-trimethyl-cyclopent-3-enyl)-hex-4-enal, (*4Z*)-6-((1R)-2,2,3-trimethyl-cyclopent-3-enyl)-hex-4-enal, 6-(2,2,3-trimethyl-cyclopentyl)-hex-4-enal, (*4Z*)-6-(2,2,3-trimethyl-cyclopentyl)-hex-4-enal, (*4E*)-6-(2,2,3-trimethyl-cyclopentyl)-hex-4-enal, 4-methyl-6-(2,2,3-trimethyl-cyclopent-3-enyl)-hex-4-enal, (*4E*)-4-methyl-6-(2,2,3-trimethyl-cyclopent-3-enyl)-hex-4-enal, (*4Z*)-4-methyl-6-(2,2,3-trimethyl-cyclopent-3-enyl)-hex-4-enal, 4-methyl-6-(2,2,3-trimethyl-cyclopentyl)-hexanal, 4-ethyl-6-(2,2,3-trimethyl-cyclopentyl)-hexanal, 3-methyl-6-(2,2,3-trimethyl-cyclopentyl)-hexanal, 2,4-dimethyl-6-(2,2,3-trimethyl-cyclopentyl)-hexanal, 7-(2,2,3-trimethyl-cyclopent-3-enyl)-hept-5-en-2-one, (*4E*)-7-(2,2,3-trimethyl-cyclopent-3-enyl)-hept-5-en-2-one, (*4Z*)-7-(2,2,3-trimethyl-cyclopent-3-enyl)-hept-5-en-2-one, 2-(6,6-diethoxy-3-ethylhexyl)-1,1,5-trimethyl-cyclopentane, 4-(6,6-diethoxy-hex-2-enyl)-1,5,5-trimethyl-cyclopentene, 4-((2*E*)-6,6-diethoxy-hex-2-enyl)-1,5,5-trimethyl-cyclopentene, 4-((2*Z*)-6,6-diethoxy-hex-2-enyl)-1,5,5-trimethyl-cyclopentene.

In another aspect, the invention relates to the use of the compounds of formula (I) as described above, and to the use of one or more isomers of 3-methyl-6-(2,2,3-trimethyl-cyclopent-3-enyl)-hex-4-enal as fragrant or flavouring agents. This invention also relates to a fragrant or flavouring composition containing at least one campholenic derivative of the invention and/or 3-methyl-6-(2,2,3-trimethyl-cyclopent-3-enyl)-hex-4-enal, which has never been described as an organoleptic compound.

This invention includes any fragrant or flavouring composition comprising one or more isomers of a compound of formula (I), and/or 3-methyl-6-(2,2,3-trimethyl-cyclopent-3-enyl)-hex-4-enal. According to a preferred embodiment, this invention relates to a composition comprising at least two or three isomers of a compound of formula (I), and/or 3-methyl-6-(2,2,3-trimethyl-cyclopent-3-enyl)-hex-4-enal.

The compounds of the invention may be used alone or in combination with other perfuming or flavouring ingredients, solvents, additives or fixatives, commonly used and that the man skilled in the art is able to choose in regard of the desired effect and the nature of the product to perfume or flavour.

In a first embodiment, the invention relates to the use of at least one campholenic derivative or composition containing them as described above in the perfumery field for the preparation of perfumed bases and concentrates, fragrances, perfumes and similar products; topic compositions; cosmetic compositions such as for example face and body creams, cleansers, facial treatments, talc powders, hair oils, shampoos, hair lotions, bath oils and salts, shower and bath gels, soaps, body anti-perspirants and deodorizers, pre-shave, shaving and post-shave creams and lotions, creams, toothpastes, mouth baths, pomades; cleaning products, such as for example softeners, detergents, air deodorizers and household cleaning supplies. Therefore, the invention also relates to a fragrant composition including at least one compound of formula (I) or one or more isomers of a compound of formula (I).

In a second embodiment, the invention relates to the use of the compounds or composition as described above, as flavouring agents for the preparation of flavouring compositions or articles, such as for example drinks, dairy products, ice creams, soups, sauces, dips, dishes, meat products, culinary assistances, salted biscuits or snacks and also beers, wines and tobaccos. Therefore, the invention also relates to a flavoured composition including at least one compound of formula (I) or one or more isomers of a compound of formula (I).

In a third embodiment, the invention relates to the use of the compounds or composition as described above, as masking agents of odours and/or flavours, and to any pharmaceutical, cosmetic or food composition containing at least one compound of formula (I) or one or more isomers of a compound of formula (I). Therefore, this invention also relates to any composition comprising at least one compound of formula (I), as herein described, in combination with any suitable excipient, especially pharmaceutical or cosmetic or dietary excipient.

The compounds of the invention may be used in a concentration comprised in a range from 0.001% to 99% in weight, preferably from 0.1% to 50% in weight, more preferably from 0.1% to 30% in weight. It is known by the man skilled in the art that these values depend of the nature of the composition/article to be perfumed and/or flavoured, the desired intensity of the perfume and/or flavour, and of the nature of the other ingredients present in said composition or article. According to a preferred embodiment of the invention, compounds are used in an olfactory effective amount.

The new derivatives of the present invention can be easily prepared by different ways with reactions known by the person of the art. For instance, derivatives of general formula (I), as above described, wherein Y is a C(O)R₆ group and wherein the double bond between C2-C3 is absent, may be prepared by a Claisen rearrangement, as shown in Scheme 1, from compounds of formula (II), wherein R1 to R6 are as described hereabove. The molecules of formula (II) can be obtained via the condensation of an appropriately substituted Grignard reagent with campholenaldehyde, saturated or not, followed by transetherification with the adequate vinyl ether or reaction with appropriate allylic halides, according to known procedures.

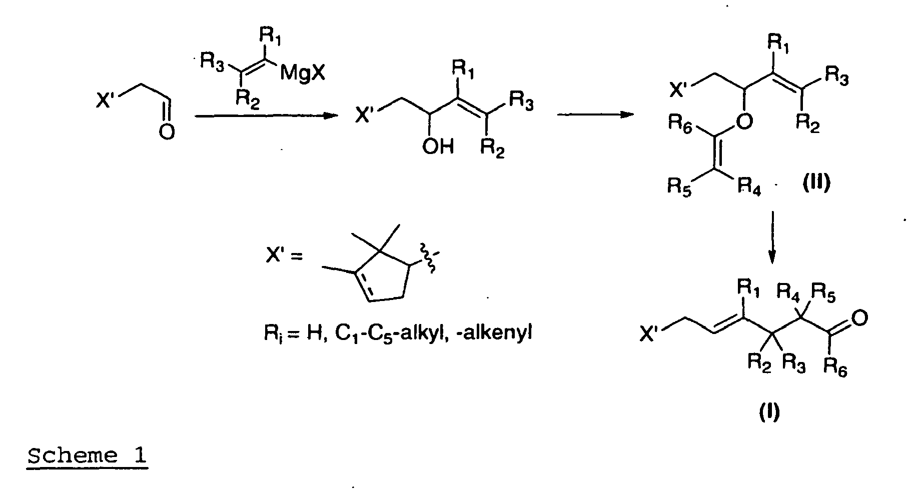

The new derivatives of formula (I), wherein R₁₋₅ have the meaning as defined in formula (I), wherein Y is a CN group, may be obtained *via* a Knoevenagel-Doebner condensation between compound of formula (III) and cyanoacetic acid or ethyl cyanoacetate, followed by reduction of one or more double bonds, as shown in Scheme 2. Compounds (III) are obtained from campholenaldehyde and appropriate oxo-compounds by crotonisation, according to procedures known in the art, and may be used directly or its α,β-insaturation may be reduced before the Knoevenagel condensation.

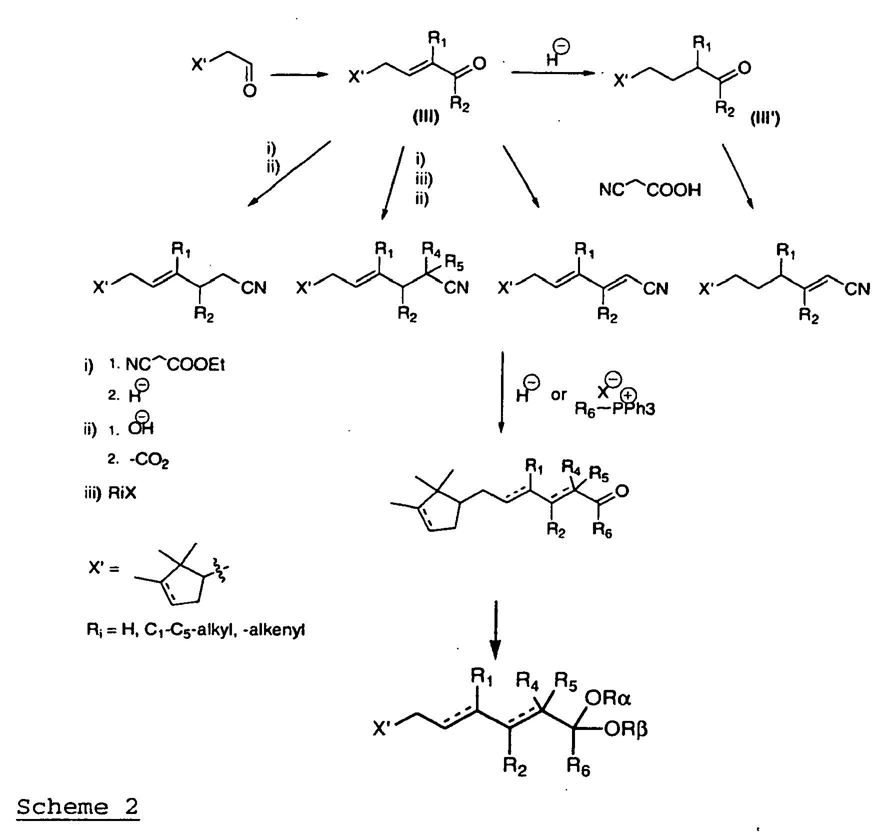

When derivatives of formula (I), with Y being a cyano group, are obtained, the compounds of formula (I) wherein Y is a C(O)R6 group, are easy to prepare, for instance via a reduction with diisobutylaluminumhydride or *via* an alkylation with phosphonium derivatives. Compounds of formula (I), wherein Y is a CR₆(ORα)(ORβ) group are then simply obtained from (I), with Y being a C(O)R₆ group via acetalisation with the appropriate alcohol or diol.

Campholenaldehyde, the starting material, is either commercial or prepared from α-pinene, *via* pinene oxide, according to known procedure. (S)-(-)Campholenic aldehyde and its (R)-(+)-enantiomer may be prepared from (1R)-(+) and (1S)-(-)-α-pinene, respectively. The double bond may be hydrogenated to prepare compounds of formula (I), as described before, where the cyclic double bond is absent.

Following examples detail the processes of preparation of the compounds of the invention and their use. These examples are presented with an only goal of illustration and shall not be regarded as limiting the scope of the invention.

### Example 1: Preparation of 6-(2,2,3-trimethyl-cyclopent-3-enyl)-hex-4-enenitrile

A suspension of (3-cyano-propyl)-triphenylphosphonium chloride (readily prepared by known reaction between triphenylphosphine and 4-chloro-butyronitrile) in toluene, with potassium carbonate, commercial campholenaldehyde and a catalytic amount of benzoic acid, was refluxed for 24 hours. The mixture was then cooled down and filtered. The filtrate was washed twice with warm water, dried over magnesium sulphate and the solvents were evaporated to give a crude oil, which was triturated with t-butyl methyl ether to precipitate most of the phosphine oxide. After filtration, the solvents were evaporated and the crude oil distilled.

The compound thus obtained is a *cis*/*trans* (90:10) mixture. Bp = 98-100°C/0.76-0.79 torr

### Cis-isomer:

**¹H-NMR** (200MHz, CDCl₃): δ 0.83 (s, 3H), 1.03 (s, 3H), 1.6-1.65 (m, 3H), 1.75-1.95 (m, 2H), 1.95-2.4 (m, 3H), 2.4-2.55 (m, 4H), 5.2-5.3 (m, 1H), 5.35-5.7 (3, 2H).
**¹³C-NMR** (50MHz, CDCl₃) : δ 13.02, 17.93, 20.13, 23.76, 26.28, 28.32, 35.93, 47.15, 50.66, 119.82, 212.99, 125.57, 133.51, 148.83.
*Trans*-isomer: selected data
**¹H-NMR** (200MHz, CDCl₃) : δ 0.80 (s, 3H), 1.01 (s, 3H). **¹³C-NMR** (50MHz, CDCl₃) : δ 18.14, 28.81, 33.69, 50.41, 122.08, 126.41, 134.25, 148.89.
IR (film, cm⁻¹): 798m, 1013w, 1360m, 1444m, 1463m, 1653w, 2246w, 2835m, 2866s, 2895s, 2930s, 2956s, 3013w, 3035w.
**MS** (EI, intensity (%)): 203 (M+, 10), 188 (100), 171 (38), 160 (19), 147 (35), 133 (19), 121 (31), 109 (83), 108 (91), 107 (26), 105 (24), 95 (26), 94 (31), 93 (44), 91 (44), 81 (19), 79 (40), 77 (33), 55 (36), 53 (27), 41 (72), 39 (26).

### Example 2: Preparation of (+)-(1'R)-(6-(2,2,3-trimethyl-cyclopent-3-enyl)-hex-4-enenitrile

It was prepared from (R)-(+)-campholenaldehyde according to example 1.
α_{D}(neat) = + 1.92°

### Example 3: Preparation of 6-(2,2,3-trimethyl-cyclopentyl)-hex-4-enenitrile

6-(2,2,3-Trimethyl-cyclopentyl)-hex-4-enenitrile was prepared from 2*H*-dihydro-campholenaldehyde and (3-cyanopropyl)-triphenylphosphonium chloride, according to example 1.

The product is obtained as a *cis*/*trans* (90:10) mixture.
Bp = 82°C/0.02 torr

### Cis-isomer:

**¹H-NMR** (200MHz, CDCl₃) : δ 0.53 (s, 3H), 0.82 (d, 3H, J = 6.7Hz), 0.88 (s, 3H), 1.05-1.25 (m, 2H), 1.25-1.55 (m, 2H), 1.55-1.90 (m, 3H), 2.05-2.25 (m, 1H), 2.27-2.47 (m, 4H), 5.20-5.40 (m, 1H), 5.45-5.65 (m, 1H).
**¹³C-NMR** (50MHz, CDCl₃) : δ 13.82, 14.33, 17.47, 23.24, 25.60, 28.13, 28.27, 29.85, 44.97, 50.89, 119.34, 124.67, 133.30.

### Trans-isomer:

**¹H-NMR** (200MHz, CDCl₃, selected data): δ 0.50 (s, 3H), 0.84 (s, 3H).
**¹³C-NMR** (50MHz, CDCl₃) : δ 12.54, 17.67, 19.65, 25.80, 27.84, 28.01, 33.69, 35.45, 50.18, 50.52, 119.34, 125.05, 133.03. **IR** (film, cm⁻¹): 1367m, 1388w, 1428m, 1453m, 1465m, 1660w, 1710w, 1727w, 2247m, 28870s, 2955s, 3013m.
**MS** (EI, intensity (%)): 205 (M+, 1), 190(7), 188(4), 162(8), 148(13), 134(8), 120(6), 111(72), 109(18), 95(24), 84(33), 70(22), 69(100), 67(25), 55(38), 41(41).

### Example 4: Preparation of 4-methyl-6-(2,2,3-trimethyl-cyclopent-3-enyl)-hex-4-enenitrile

To a suspension of (3-cyano-propyl)-triphenylphosphonium chloride (see Example 1) in tetrahydrofurane was added portionwise one molar equivalent of potassium tert-butoxide. After stirring at room temperature for 30 minutes, the mixture was cooled down (ice bath) before slowly adding methyl iodide. The mixture was further stirred for 1 hour and another one equivalent of potassium tert-butoxide was added portionwise. After stirring at room temperature for another hour, campholenaldehyde was added and the reaction mixture was further stirred overnight. The reaction mixture was poured into water:t-butyl methyl ether (50:50). The separated aqueous phase was acidified and extracted three times with t-butyl methyl ether. The combined organic layers were washed twice with water, then with saturated aqueous sodium bicarbonate and with brine. After drying over magnesium sulphate, the solvents were evaporated and the crude product purified by distillation.
Bp = 104-106°C/0.7-0.8 torr
The compound is obtained as a *cis*/*trans* (50:50) mixture.
**¹H-NMR** (200MHz, CDCl₃): δ 0.79 (s, 3H), 0.99 (s, 3H), 1.58-1.63 (m, 3H), 1.63-2.53 (m, 12H), 2.41 (s, 3H), 5.2-5.25 (m, 1H), 5.25-5.38 (m, 1H).
**¹³C-NMR** (50MHz, CDCl₃): δ 12.57, 15.64 & 15.84, 16.31 & 22.72, 19.68, 25.84 & 25.87, 27.56, 28.36 & 28.5, 35.07, 35.48 & 35.52, 46.68, 50.34 & 50.53, 119.47 & 119.52, 121.56 & 121.69, 127.43 & 128.48, 130.79 & 130.9, 148.41 & 148.49.
**IR** (film, cm⁻¹): 799m, 1013w, 13560m, 1382w, 1445m, 1462m, 1691w, 2246w, 2835m, 2866m, 2930s, 2956s, 3036w.
**MS** (EI, intensity (%), *cis* isomer): 217 (M+, 12), 202 (19), 185 (13), 146 (12), 122 (45), 121 (93), 109 (100), 108 (93), 107 (83), 95 (31), 93 (48), 91 (48), 81 (63), 79 (54), 77 (35), 67 (76), 55 (37), 53 (34), 41 (66), 39 (23).
**MS** (EI, intensity (%), *trans* isomer): 217 (M+, 15), 202 (27), 185 (5), 146 (10), 122 (54), 121 (100), 109 (99), 108 (100), 107 (90), 95 (33), 93 (48), 91 (50), 81 (64), 79 (53), 77 (35), 67 (75), 55 (35), 53 (33), 41 (61), 39 (22).

### Example 5: Preparation of 4-methyl-6-(2,2,3-trimethyl-cyclopentyl)-hexanenitrile

2-Methyl-4-(2,2,3-trimethyl-cyclopent-3-enyl)-but-2-enal (obtained from known procedure) was condensed with cyanoacetic acid in the presence of 2,6-lutidine at room temperature to afford 4-methyl-6-(2,2,3-trimethyl-cyclopent-3-enyl)-hexa-2,4-dienenitrile. After removal of the heavy by-products, the crude 4-methyl-6-(2,2,3-trimethyl-cyclopent-3-enyl)-hexa-2,4-dienenitrile was hydrogenated under H₂ pressure (pH₂ = 20 bars ; room temperature) to give 4-methyl-6-(2,2,3-trimethyl-cyclopentyl)-hexanenitrile.
Bp = 95°C/0.07 torr
**¹H-NMR** (200MHz, CDCl₃) : δ 0.49 (s, 3H), 0.82 (d, 3H, *J* = 6.7 Hz), 0.85 (s, 3H), 0.91 (2 d, 3H, *J* = 6.3 Hz), 1.0-1.8 (m, 13H), 2.25-2.42 (m, 2H).
**¹³C-NMR** (50MHz, CDCl₃) : δ 13.91 & 14.43, 14.93 & 15.0, 18.77 & 19.06, 25.68 & 25.72, 27.5 & 27.69, 28.24 & 28.38, 30.13, 32.1 & 32.27, 32.55, 35.35 & 35.65, 42.32, 45.24, 50.85 & 51.17.
**IR** (film, cm⁻¹): 1365m, 1385w, 1427w, 1465m, 2246w, 2869s, 2955s.
**MS** (EI, intensity (%)): 221 (M+, 3), 206 (6), 204 (9), 178 (18), 84 (100), 83 (26), 70 (56), 69 (83), 55 (58), 41 (45).

### Example 6: Preparation of 4-ethyl-6-(2,2,3-trimethyl-cyclopentyl)-hexanenitrile

4-Ethyl-6-(2,2,3-trimethyl-cyclopentyl)-hexanenitrile was prepared from 2-ethyl-4-(2,2,3-trimethyl-cyclopent-3-enyl)-but-2-enal (prepared from campholenaldehyde and propanal, via known procedure) and cyanoacetic acid according to example 5.
The compound is obtained as a mixture of isomers (70:30). Bp = 112°C/0.03 torr
**¹H-NMR** (200MHz, CDCl₃, common protons): δ 0.46 (s, 3H), 0.7-0.9 (m, 9H), 0.9-1.5 (m, 12H), 1.5-1.85 (m, 3H).
Major isomers: δ 2.27 (t, 2H, *J* = 7.4 Hz); minor isomers: δ 2.54 (t, 2H, *J* = 7.2 Hz).
**¹³C-NMR** (50MHz, CDCl₃) :
Major isomers: δ 10.18 & 10.52, 13.73 & 13.76, 14.27, 14.52 & 14.66, 24.72 & 25.16, 25.54 & 25.58, 27.0 & 27.27, 28.19 & 28.23, 28.58 & 28.83, 29.97 & 30.04, 31.31 & 31.39, 38.05 & 38.25, 42.12 & 42.15, 45.08 & 45.11, 50.93 & 51.14, 119.89.
Minor isomers (selected data): δ 10.55 & 10.88, 25.46 & 25.92, 26.97 & 27.05, 30.68 & 31.02, 39.12 & 39.23, 50.99. IR (film, cm⁻¹): 1118w, 1244w, 1367m, 1384w, 1461m, 1738w, 2247w, 2869s, 2956s.
**MS** (EI, intensity (%)): 235 (M+, 1), 220 (3), 192 (10), 84 (162), 83 (26), 70 (67), 69 (89), 55 (49), 41 (43).

### Example 7: Preparation of 4-methyl-6-(2,2,3-trimethyl-cyclopentyl)-hex-3-enenitrile

2-Methyl-4-(2,2,3-trimethyl-cyclopentyl)-butyraldehyde (easily obtained from 2-methyl-4-(2,2,3-trimethyl-cyclopent-3-enyl)-but-2-enal) was condensed with cyanoacetic acid in the presence of 2,6-lutidine at room temperature to afford 4-methyl-6-(2,2,3-trimethyl-cyclopentyl)-hex-3-enenitrile, as a (40:60) mixture of isomers.
Bp = 95°C/0.03 torr
**¹H-NMR** (200MHz, CDCl₃) : δ 0.49 (s, 3H), 0.75-0.9 (m, 6H), 0.9-1.4 (m, 5H), 1.4-1.6 (m, 2H), 1.6-2.1(m, 3H), 1.65 (s, 3H), 3.03 (d, 2H, *J* = 6.9 Hz), 5.1-5.2 (m, 1H).
Minor isomer (selected data): δ 1.73 (d, 3H, *J* = 1.3 Hz).
**¹³C-NMR** (50MHz, CDCl₃) :
Major isomer: δ 13.82, 14.37, 16.16, 16.39, 25.56, 28.04, 28.46, 30.05, 38.4, 42.25, 45.11, 50.29, 111.11, 118.63, 142.96.
Minor isomer: δ 13.82, 14.37, 16.02, 16.39, 23.13, 28.15, 28.25, 28.46, 31.13, 38.4, 42.31, 45.19, 50.61, 111.72, 118.63, 143.09.
**IR** (film, cm⁻¹): 920w, 1049w, 1366m, 1386m, 1418w, 1453m, 1466m, 1669w, 1754w, 2250w, 2869s, 2954s.
MS (EI, intensity (%)): 219(M+,1), 204(5), 179(8), 124(9), 109(26), 95(16), 84(67), 83(32), 81(17), 70(44), 69(100), 67(26), 55(40), 41(49).
219(M+,1), 204(7), 124(15), 109(69), 95(23), 84(49), 83(26), 82(36), 81(20), 70(35), 69(100), 67(30), 55(39), 41(48).

### Example 8: Preparation of 6-(2,2,3-trimethyl-cyclopent-3-enyl)-hex-4-enal

A 1M solution of diisobutylaluminium hydride in toluene was added dropwise to a solution of 6-(2,2,3-trimethyl-cyclopent-3-enyl)-hex-4-enenitrile at such a rate that the temperature remains under 40°C. After the addition, the solution was heated at 80°C for 2 hours. The hydrolysis was carefully performed pouring the reaction solution into a mixture of acetic acid and ice. The aqueous phase was extracted twice with t-butyl methyl ether and the combined organic phases were washed twice with water, with an aqueous saturated solution of sodium bicarbonate and with brine. After drying over magnesium sulphate, the solvents were evaporated to give a crude oil. The purification consisted in a bulb-to-bulb evaporation prior to distillation.
Bp = 76-78°C/0.2 torr
**¹H-NMR** (200MHz, CDCl₃) :
*Cis*-isomer: δ 0.8 (s, 3H), 1.1 (s, 3H), 1.55-1.65 (m, 3H), 1.65-2.6 (m, 9H), 5.15-5.25 (m, 1H), 5.25-5.55 (m, 2H), 9.78 (t, 1H, *J* = 1.5 Hz).
*Trans*-isomer: (selected data) δ 0.76 (s, 3H), 0.97 (s, 3H), 9.66 (m, 1H).
**¹³C-NMR** (50MHz, CDCl₃): (cis isomer) δ 12.58, 19.67, 20.11, 25.85, 27.77, 35.52, 43.76, 46.69, 50.31, 121.6, 127.2, 131.09, 148.49, 202.15.
**IR** (film, cm⁻¹): 798m, 1013w, 1360m, 1384w, 1409w, 1444m, 1463m, 1727s, 2719w, 2833m, 2866m, 2894s, 2930s, 2955s, 3010m, 3035w.
MS (EI, intensity (%)): 206 (M+, 8), 191 (19), 173 (12), 147 (28), 121 (30), 109 (48), 108 (100), 107 (45), 105 (20), 97 (21), 95 (36), 93 (52), 91 (36), 81 (27), 79 (65), 77 (30), 69 (36), 67 (76), 55 (46), 53 (21), 43 (24), 41 (80), 39 (26).

### Example 9: Preparation of (+)-(1'R)-6-(2,2,3-trimethyl-cyclopent-3-enyl)-hex-4-enal

It was prepared from nitrile of example 2, according to example 8.
α_{D} (neat) = + 2°

### Example 10: Preparation of 6-(2,2,3-trimethyl-cyclopentyl)-hex-4-enal

6-(2,2,3-Trimethyl-cyclopentyl)-hex-4-enal was obtained from 6-(2,2,3-Trimethyl-cyclopentyl)-hex-4-enenitrile, according to example 8.

The product is obtained as a *cis*/*trans* mixture (90:10).

### Cis-isomer:

**¹H-NMR** (200MHz, CDCl₃): δ 0.51 (s, 3H), 0.80 (d, 3H, *J* = 6.7Hz), 0.86 (s, 3H), 1.05-1.25 (m, 2H), 1.25-1.90 (m, 5H), 2.02-2.20 (m, 1H), 2.25-2.52 (m, 4H), 5.12-5.50 (m, 2H), 9.74 (t, 1H, *J* = 1.5Hz).
**¹³C-NMR** (50MHz, CDCl₃) : δ 13.82, 14.24, 20.03, 25.60, 28.15, 29.87, 42.18, 43.74, 44.99, 50.97, 126.75, 131.28, 202.01.

### Trans-isomer:

**¹H-NMR** (200MHz, CDCl₃, selected data): δ 0.47 (s, 3H), 0.97 (s, 3H), 9.72 (t, 1H, *J* = 1.6Hz).
**¹³C-NMR** (50MHz, CDCl₃) : 12.52, 13.24, 25.13, 25.79, 27.72, 35.48, 42.09, 43.41, 45.10, 50.28, 127.16, 130.99, 201.92. **IR** (film, cm⁻¹) : 1366m, 1388m, 1409w, 1453m, 1466m, 1728s, 2719m, 2870s, 2955s, 3010m.
MS (EI, intensity (%)): 208(M+, 1), 193(3), 124(6), 111(33), 109(25), 95(28), 80(25), 79(22), 69(100), 67(24), 55(38), 41(40).

### Example 11: Preparation of 4-methyl-6-(2,2,3-trimethyl-cyclopent-3-enyl)-hex-4-enal

4-Methyl-6-(2,2,3-trimethyl-cyclopent-3-enyl)-hex-4-enal was prepared from 4-methyl-6-(2,2,3-trimethyl-cyclopent-3-enyl)-hex-4-enenitrile according to example 8, as a (60:40) *cis*/*trans* mixture.
**¹H-NMR** (200MHz, CDCl₃):
*Cis*-isomer: δ 0.77 (s, 3H), 0.97 (s, 3H), 1.55-1.65 (m, 3H), 1.65-1.70 (m, 3H), 1.70-2.05 (m, 4H), 2.05-2.2.5 (m, 2H), 2.25-2.55 (m, 3H), 5.15-5.25 (m, 2H), 9.77 (t, 1H, *J* = 1.7 Hz).
*Trans*-isomer: (selected data) δ 1.57-1.60 (m, 3H), 9.74 (t, 1H, *J* = 1.9 Hz).
**¹³C-NMR** (50MHz, CDCl₃) :
*Cis*-isomer: δ 12.57 & 19.67, 23.09, 24.28, 25.86, 28.31, 35.60, 42.25, 46.66, 50.66, 121.63, 126.41, 132.67, 148.50, 202.28.
*Trans*-isomer: δ 12.57 & 19.67, 16.13, 25.88, 28.45, 31.96, 35.53, 42.13, 46.66, 50.51, 121.68, 125.35, 132.74, 148.50, 202.61.

### Example 12: Preparation of 4-methyl-6-(2,2,3-trimethyl-cyclopentyl)-hexanal

4-Methyl-6-(2,2,3-trimethyl-cyclopentyl)-hexanal was prepared from 4-methyl-6-(2,2,3-trimethyl-cyclopentyl)-hexanenitrile according to example 8.
**¹H-NMR** (200MHz, CDCl₃) : δ 0.49 (s, 3H), 0.74-0.91 (m, 6H), 0.82 (d, 3H, *J* = 6.7 Hz), 0.91-1.56 (m, 10H), 1.56-1.85 (m, 4H), 2.35-2.5 (3, 2H), 9.78 (t, 1H, *J* = 1.9 Hz).
**¹³C-NMR** (50MHz, CDCl₃) : δ 13.92 & 14.42, 19.29 & 19.6, 25.71 & 25.74, 27.66 & 27.85, 28.28 & 28.42, 28.69 & 29.21, 30.17, 32.68 & 32.93, 35.8 & 36.08, 41.71 & 41.83, 42.31, 45.27, 50.95 & 51.26, 203.04.
**IR** (film, cm⁻¹): 1365w, 1387w, 1465m, 1729s, 2714w, 2869s, 2931s, 2955s.
**MS** (EI, intensity (%)): 224 (M+, 2), 207 (6), 163 (9), 110 (20), 109 (36), 95 (34), 85 (33), 84 (70), 83 (34), 81 (29), 70 (79), 69 (100), 67 (23), 57 (19), 55 (66), 43 (22), 41 (55).

### Example 13: Preparation of 4-ethyl-6-(2,2,3-trimethyl-cyclopentyl)-hexanal

4-Ethyl-6-(2,2,3-trimethyl-cyclopentyl)-hexanal was prepared from 4-ethyl-6-(2,2,3-trimethyl-cyclopentyl)-hexanenitrile according to example 8. The compound was purified by column chromatography and consists in a (50:50) mixture of isomers.
¹H-NMR (200MHz, CDCl₃): δ 0.45 (s, 3H), 0.7-0.95 (m, 9H), 0.78 (d, 3H, *J*=6.7 Hz), 0.95-1.35 (m, 12H), 1.35-1.65 (m, 3H), 1.65-1.85 (m, 3H), 2.34 & 2.38 (2 td, 2H, *J* = 1.7 & 1.5 Hz, *J* = 7.8 & 7.6 Hz), 9.73 (t, 1H, *J* = 1.9 Hz).
**¹³C-NMR** (50MHz, CDCl₃) : δ 10.45 & 10.77, 13.78, 14.30, 24.93 & 25.68, 25.24, 25.58, 27.19 & 27.21, 28.24, 30.03, 31.87 & 31.90, 38.55 & 38.68, 41.20 & 41.42, 42.18, 45.14, 51.07 & 51.11, 202.98.
**IR** (film, cm⁻¹): 1051m, 1103m, 1367m, 1385m, 1461s, 1728s, 2715w, 2869s, 2955s.
**MS** (EI, intensity (%)): 238 (M+,1), 223(4), 209(4), 177(6), 123(8), 110(20), 109(27), 99(19), 95(27), 84(66), 83(37), 81(26), 70(79), 69(100), 67(23), 57(19), 55(81), 43(30), 41(65).

### Example 14: Preparation of 3-methyl-6-(2,2,3-trimethyl-cyclopentyl)-hexanal

3-Methyl-6-(2,2,3-trimethyl-cyclopentyl)-hexanal was obtained, following the procedure of example 8, from a nitrile prepared according to example 5 from 5-(2,2,3-trimethyl-cyclopent-3-enyl)-pent-3-en-2-one (prepared from campholenaldehyde and acetone).

The crude compound was purified by column chromatography.
**¹H-NMR** (200MHz, CDCl₃) : δ 0.47 (s, 3H), 0.80 (d, 1H, *J* = 6.7Hz), 0.83 (s, 3H), 0.94 (2d, 1H, *J* = 6.6 Hz), 1.0-1.55 (m, 10H), 1.55-1.85 (m, 2H), 2.03 (o, 1H, *J* = 6.5 Hz), 2.18 & 2.24 (td, 2H, *J* = 2.2 Hz, *J* = 7.7 Hz) & 2.34 & 2.42 (td, 2H, *J* = 2 Hz, *J* = 5.8 Hz), 9.74 (t, 1H, *J* = 2.2 Hz).
**¹³C-NMR** (50MHz, CDCl₃) : δ 13.85, 14.31, 19.86 & 20.05, 25.63, 26.14 & 26.22, 28.12 & 28.25, 28.21, 30.12, 30.47 & 30.58, 37.28 & 37.39, 42.20, 45.19, 20.79 & 50.85, 50.99 & 51.17, 202.99 & 203.02.
**IR** (film, cm⁻¹): 1127w, 1367m, 1384m, 1464s, 1728s, 2712m, 2869s, 2954s.
MS (EI, intensity (%)): 224(M+, 1), 109(13), 95(21), 84(61), 83(28), 81(22), 70(86), 69(100), 67(18), 55(52), 43(23), 41(51).

### Example 15: Preparation of 2,4-dimethyl-6-(2,2,3-trimethyl-cyclopentyl)-hexanal

Aldehyde from example 12 was alkylated with methyl iodide, according to known procedure, via its diisobutylenamine, obtained with diisobutylamine through azeotropic water removal in toluene in the presence of catalytic amount of PTSA.

The obtained product was purified by column chromatography, it consists in a mixture of isomers, 2 observable groups in NMR, ratio (60:40), composed each of 2 diastereoisomers.

### Major isomers:

**¹H-NMR** (200MHz, CDCl₃) : δ 0.48 (s, 3H), 0.80 (d, 3H, *J* = 6.7 Hz), 0.83 (s, 3H), 0.87 (d, 3H, *J* = 6.5 Hz), 0.92-1.51 (m, 10H), 1.06 (d, 3H, *J* = 6.9 Hz), 1.51-1.87 (m, 3H), 9.55 (d, 1H, *J* = 1.6 Hz), 9.57 (d, 1H, *J* = 1.5 Hz).
**¹³C-NMR** (50MHz, CDCl₃) : δ 13.85, 14.10 & 14.26, 14.35, 19.66 & 19.99, 25.64, 27.63, 28.20, 30.10, 30.51 & 30.95, 35.73 & 36.02, 38.17 & 38.51, 42.24, 44.13, 45.20, 50.83 & 51.17, 205.40.

### Minor isomers:

**¹H-NMR** (200MHz, CDCl₃, selected data): δ 0.88 (d, 3H, *J* = 6.6 Hz), 1.05 (d, 3H, *J* = 7.0 Hz), 9.59 (2d, 1H, *J* = 2.0 Hz).
**¹³C-NMR** (50MHz, CDCl₃) : δ 13.24 & 13.38, 13.85, 14.35, 19.10 & 19.43, 25.68, 27.42, 28.34, 30.10, 30.41 & 30.60, 36.40 & 36.67, 37.33 & 37.92, 42.24, 44.23, 45.20, 50.88 & 51.14, 205.35.
**IR** (film, cm⁻¹): 1367w, 1380w, 1464m, 1709m, 1729s, 2704w, 2870s, 2956s.
MS (EI, intensity (%)): 238 (M+), 223 (1), 180 (6), 125 (15), 109 (32), 99 (22), 97 (22), 95 (27), 84 (62), 83 (37), 81 (21), 71 (20), 70 (73), 69 (100), 57 (21), 55 (72), 43 (39), 41 (57).

### Example 16: Preparation of 7-(2,2,3-trimethyl-cyclopent-3-enyl)-hept-5-en-2-one

7-(2,2,3-Trimethyl-cyclopent-3-enyl)-hept-5-en-2-one was prepared from nitrile of example 1 according to known procedure. It is obtained as a (95:5) *cis*/*trans* mixture.
Bp = 76-78°C/0.04 torr
**¹H-NMR** (200MHz, CDCl₃) : δ 0.78 (s, 3H), 0.98 (s, 3H), 1.55-1.65 (m, 3H), 1.65-1.9 (m, 2H), 1.9-2.4 (m, 5H), 2.13 (s, 3H), 2.4-2.55 (m, 2H), 5.15-5.25 (m, 1H), 5.25-5.5 (m, 2H).
**¹³C-NMR** (50MHz, CDCl₃) : δ 12.59, 19.69, 21.71, 25.86, 27.73, 29.92, 35.52, 43.55, 46.69, 50.38, 121.61, 127.75, 130.65, 148.51, 208.43.
**IR** (film, cm⁻¹): 578w, 798m, 1013w, 1161m, 1361s, 1412m, 1438s, 1462s, 1654w, 1719s, 2836m, 2867s, 2957s, 3009m, 3036m.
**MS** (EI, intensity (%)): 220(M+,2), 205(3), 187(4), 162(5), 150(8), 147(38), 121(17), 109(34), 108(86), 107(24), 93(51), 91(31), 81(20), 79(36), 77(24), 67(41), 55(22), 43(100), 41(28).

### Example 17: Preparation of 2-(6,6-diethoxy-3-ethyl-hexyl)-1,1,5-trimethyl-cyclopentane

2-(6,6-Diethoxy-3-ethyl-hexyl)-1,1,5-trimethyl-cyclopentane was prepared by treating compound of example 13 with ethanol, in presence of triethylorthoformate with catalytic amount of PTSA. The crude product was pure enough not to be further purified. It consists in a (50:50) mixture of isomers.
**¹H-NMR** (200MHz, CDCl₃): δ 0.47 (s, 3H), 0.75-0.9 (m, 3H), 0.8 (d, 3H, *J* = 6.7 Hz), 0.83 (s, 3H), 0.9-1.37 (m, 10H), 1.19 (t, 6H, *J* = 7.1 Hz), 1.37-1.67 (m, 3H), 1.67-1.85 (m, 2H), 3.56 (m, 4H), 4.45 (t, 1H, *J* = 5.7 Hz).
**¹³C-MMR** (50MHz, CDCl₃) : δ 10.68 & 10.99, 13.87, 14.37, 15.33, 25.58 & 26.02, 25.67, 27.36 & 27.41, 27.64 & 28.01, 28.33, 30.15, 30.47 & 30.74, 32.16 & 32.20, 38.98 & 39.05, 42.25, 51.23, 60.67, 103.32.
**IR** (film, cm⁻¹): 1016m, 1064s, 1126s, 1345w, 1375m, 1460m, 2870s, 2956s.
**MS** (EI, intensity (%)): 312 (M+, 1), 268(1), 103(100), 95(9), 75(31), 69(22), 55(15), 47(14), 41(9).

### Example 18: Preparation of 4-(6,6-diethoxy-hex-2-enyl)-1,5,5-trimethyl-cyclopentene

4-(6,6-Diethoxy-hex-2-enyl)-1,5,5-trimethyl-cyclopentene was prepared according to example 17, starting from aldehyde of example 8.
Bp = 94°C/0.07 torr

### Cis-isomer:

¹H-NMR (200MHz, CDCl₃) : δ 0.77 (s, 3H), 0.97 (s, 3H), 1.17 (t, 6H, *J* = 7.1), 1.50-1.90 (m, 7H), 1.90-2.35 (m, 5H), 3.54 (m, 4H), 4.46 (t, 1H, *J* = 5.7), 5.15-5.25 (m, 1H), 5.25-5.47 (m, 2H).
¹³C-NMR (50MHz, CDCl₃) : δ 12.51, 15.28, 19.63, 22.57, 25.82, 27.69, 33.46, 35.49, 46.65, 50.45, 60.84, 102.33, 121.67, 128.84, 129.89, 148.37.

### Trans-isomer: selected data

¹H-NMR (200MHz, CDCl₃) : δ 0.74 (s, 3H), 0.95 (s, 3H).
¹³C-MMR (50MHz, CDCl₃) : δ 129.60, 130.42.
IR (film, cm⁻¹): 798w, 1014w, 1064s, 1130s, 1360w, 1375m, 1444m, 1744w, 2871s, 1931s, 2957s, 3007m, 3037w.

### Example 19: Olfactive description of the campholenic derivatives of the invention

The odours of the compounds of the invention have been described by a professional evaluation panel and are gathered in table 1 hereinafter.

**Table 1:**

| Example | R₁ | R₂ | R₃ | R₄ | R₅ | Y | Double bonds | Odour description |
|---|---|---|---|---|---|---|---|---|
| 1-2 | H | H | H | H | H | CN | C3-C4 C3'-C4' | Floral, slightly woody (cedarwood) |
| 4 | CH₃ | H | H | H | H | CN | C3-C4 C3'-C4' | Fruity, slightly anisic |
| 8-9 | H | H | H | H | H | CHO | C3-C4 C3'-C4' | Floral (lily of the valley), green, very powerful |
| 11 | CH₃ | H | H | H | H | CHO | C3-C4 C3'-C4' | Floral (lily of the valley), Lilial®, Lyral® |
| 16 | H | H | H | H | H | C(O)CH₃ | C3-C4 C3'-C4' | Green (Fig leaves), sweet |
| 18 | H | H | H | H | H | CH (OCH₂CH₃)₂ | C3-C4 C3'-C4' | Plastic, not powerful |
| 3 | H | H | H | H | H | CN | C3-C4 | Not powerful, floral (Helional®, Floralozone®) |
| 10 | H | H | H | H | H | CHO | C3-C4 | Powerful, floral (Lily of the valley), green |
| 7 | | H | - | H | H | CN | C2-C3 | Lactonic, woody, a bit animalic |
| 5 | CH₃ | H | H | H | H | CN | None | Sweet, gourmand, not powerful |
| 6 | CH₃ CH₂CH₃ | H | H | H | H | CN | None | Iris, carrot, powdery, plastic |
| 12 | CH₃ | H | H | H | H | CHO | None | Green, lactonic (wax), plastic, slightly woody |
| 15 | CH₃ | H | H | CH₃ | H | CHO | None | None |
| 13 | CH₂CH₃ | H | H | H | H | CHO | None | Sweet (furaneol), animal (costus, goat), a bit metallic |
| 14 | H | CH₃ | H | H | H | CHO | None | Marine, ozonic, transparent (Melonal®) |
| 17 | CH₂CH₃ | H | H | H | H | CH(OCH₂CH₃)₂ | None | Fruity (apple), alcoholic, animal, cresolic |

### Example 20: Fragrance composition containing 6-(2,2,3-trimethyl-cyclopent-3-enyl)-hex-4-enal

A lily of the valley type accord was prepared as described from the following ingredients:

| | | |
|---|---|---|
| Benzyl acetate | 10 | 10 |
| Cinnamic alcohol | 20 | 20 |
| Lemon ess. | 10 | 10 |
| Citronellol | 65 | 65 |
| Citronellyl acetate | 100 | 100 |
| Geraniol | 90 | 90 |
| *Cis*-3-Hexenol | 15 | 15 |
| *Cis*-3-Hexenyl acetate | 5 | 5 |
| Indol | 5 | 5 |
| Lemarome® | 10 | 10 |
| Methyl heptenone, 10% MIP | 10 | 10 |
| Nerol | 10 | 10 |
| Phenylethyl alcohol | 100 | 100 |
| 6-(2,2,3-Trimethyl-cyclopent-3-enyl)-hex-4-enal | - | 50 |
| DPG | 550 | 500 |

These 2 compositions were used in a shampoo base at usual dilution, known from the person of the art, and the samples containing the compound of formula (I) showed a greener note, vegetables-type, a bit mushroom, imparting very natural aspect to the lily of the valley note.

## Claims

1. Campholenic derivatives of general formula (I) 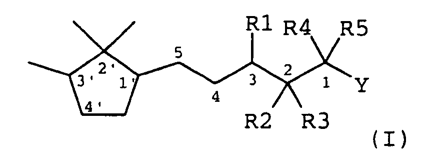 wherein
• R1, R2, R3, R4 and R5 represent, each independently, a hydrogen atom or a linear or branched C₁-C₅ alkyl or C₂-C₅ alkenyl group,
• Y represents a CN, a C(O)R₆ or a CR₆(OR_{α}) (OR_{β}) group,
wherein R₆ represents a hydrogen atom or a linear or branched C₁-C₅ alkyl or C₂-C₅ alkenyl group,
and R_{α} and R_{β} represent simultaneously a linear or branched C₁, C₂, C₃, C₄ or C₅ alkyl or C₂, C₃, C₄ or C₅ alkenyl group and can form together a cycle,
• the 5-membered ring is saturated or has a double bond between C3' and C4' in formula (I), and
• the side chain, optionally, has a double bond between C1 and C2 and/or between C3 and C4,
• Provided that the derivative is not 3-methyl-6-(2,2,3-trimethyl-cyclopent-3-enyl)-hex-4-enal.

2. Campholenic derivatives, according to Claim 1, of general formula (Ia), 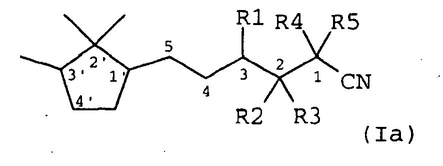 wherein
• R1, R2, R3, R4 and R5 represent, each independently, a hydrogen atom or a linear or branched C₁-C₅ alkyl or C₂-C₅ alkenyl group,
• the 5-membered ring is saturated or has a double bond between C3' and C4' in formula (Ia), and
• the side chain is optionally unsaturated between C1 and C2 and/or between C3 and C4 in formula (Ia),

3. Campholenic derivatives, according to claim 1, of general formula (Ib), 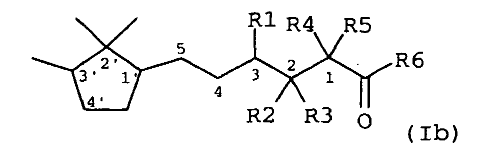 wherein
• R1, R2, R3, R4 and R5 represent, each independently, a hydrogen atom or a linear or branched C₁-C₅ alkyl or C₂-C₅ alkenyl group,
• the 5-membered ring is saturated or has a double bond between C3' and C4',
• the side chain is optionally unsaturated between C1 and C2 and/or between C3 and C4 in formula (Ib), and
• R₆ represents a hydrogen atom, a linear or branched C₁, C₂, C₃, C₄ or C₅ alkyl or C₂, C₃, C₄ or C₅ alkenyl group, preferably, R₆ is a hydrogen atom or a methyl group, more preferably R₆ is a hydrogen atom,
• Provided that the derivative is not 3-methyl-6-(2,2,3-trimethyl-cyclopent-3-enyl)-hex-4-enal.

4. Campholenic derivatives, according to claim 1, of general formula (Ic), 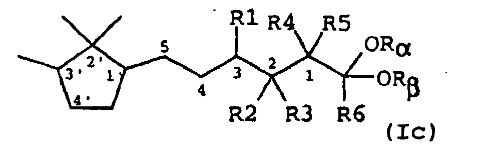 wherein
• R1, R2, R3, R4 and R5 represent, each independently, a hydrogen atom or a linear or branched C₁-C₅ alkyl or C₂-C₅ alkenyl group,
• the 5-membered ring is saturated or has a double bond between C3' and C4' in formula (IC),
• the side chain is optionally unsaturated between C1 and C2 and/or between C3 and C4 in formula (IC), and
• R_{α} and R_{β} represent simultaneously a linear or branched C₁, C₂, C₃, C₄ or C₅ alkyl or C₂, C₃, C₄ or C₅ alkenyl group, preferably, R_{α} and R_{β} are simultaneously a methyl or an ethyl group,
• R_{α} and R_{β} can form together a cycle, preferably a 5-membered ring or a 6-membered ring.

5. Campholenic derivatives, according to Claim 1, wherein said derivatives are selected in the group comprising:
6-(2,2,3-trimethyl-cyclopent-3-enyl)-hex-4-enenitrile, (*4E*)-6-(2,2,3-trimethyl-cyclopent-3-enyl)-hex-4-enenitrile, (*4Z*)-6-(2,2,3-trimethyl-cyclopent-3-enyl)-hex-4-enenitrile, (+)-6-((1R)-2,2,3-trimethyl-cyclopent-3-enyl)-hex-4-enenitrile, (-)-6-((1S)-2,2,3-trimethyl-cyclopent-3-enyl)-hex-4-enenitrile, (*4E*)-6-((1R)-2,2,3-trimethyl-cyclopent-3-enyl)-hex-4-enenitrile, (*4Z*)-6-((1R)-2,2,3-trimethyl-cyclopent-3-enyl)-hex-4-enenitrile, (*4E*)-6-((1S)-2,2,3-trimethyl-cyclopent-3-enyl)-hex-4-enenitrile, (*4Z*)-6-((1S)-2,2,3-trimethyl-cyclopent-3-enyl)-hex-4-enenitrile, 6-(2,2,3-trimethyl-cyclopentyl)-hex-4-enenitrile, (*4E*)-6-(2,2,3-trimethyl-cyclopentyl)-hex-4-enenitrile, (*4Z*)-6-(2,2,3-trimethyl-cyclopentyl)-hex-4-enenitrile, 4-methyl-6-(2,2,3-trimethyl-cyclopent-3-enyl)-hex-4-enenitrile, (*4Z*)-4-methyl-6-(2,2,3-trimethyl-cyclopent-3-enyl)-hex-4-enenitrile, (*4E*)-4-methyl-6-(2,2,3-trimethyl-cyclopent-3-enyl)-hex-4-enenitrile, 4-methyl-6-(2,2,3-trimethyl-cyclopentyl)-hexanenitrile, 4-ethyl-6-(2,2,3-trimethyl-cyclopentyl)-hexanenitrile, 4-methyl-6-(2,2,3-trimethyl-cyclopentyl)-hex-3-enenitrile, 6-(2,2,3-trimethyl-cyclopent-3-enyl)-hex-4-enal, (*4E*)-6-(2,2,3-trimethyl-cyclopent-3-enyl)-hex-4-enal, (*4Z*)-6-(2,2,3-trimethyl-cyclopent-3-enyl)-hex-4-enal, (+)-6-((1R)-2,2,3-trimethyl-cyclopent-3-enyl)-hex-4-enal, (-)-6-((1S)-2,2,3-trimethyl-cyclopent-3-enyl)-hex-4-enal, (*4Z*)-6-((1R)-2,2,3-trimethyl-cyclopent-3-enyl)-hex-4-enal, (*4Z*)-6-((1S)-2,2,3-trimethyl-cyclopent-3-enyl)-hex-4-enal, (*4Z*)-6-((1S)-2,2,3-trimethyl-cyclopent-3-enyl)-hex-4-enal, (*4Z*)-6-((1R)-2,2,3-trimethyl-cyclopent-3-enyl)-hex-4-enal, 6-(2,2,3-trimethyl-cyclopentyl)-hex-4-enal, (*4Z*)-6-(2,2,3-trimethyl-cyclopentyl)-hex-4-enal, (*4E*)-6-(2,2,3-trimethyl-cyclopentyl)-hex-4-enal, 4-methyl-6-(2,2,3-trimethyl-cyclopent-3-enyl)-hex-4-enal, (*4E*)-4-methyl-6-(2,2,3-trimethyl-cyclopent-3-enyl)-hex-4-enal, (*4Z*)-4-methyl-6-(2,2,3-trimethyl-cyclopent-3-enyl)-hex-4-enal, 4-methyl-6-(2,2,3-trimethyl-cyclopentyl)-hexanal, 4-ethyl-6-(2,2,3-trimethyl-cyclopentyl)-hexanal, 3-methyl-6-(2,2,3-trimethyl-cyclopentyl)-hexanal, 2,4-dimethyl-6-(2,2,3-trimethyl-cyclopentyl)-hexanal, 7-(2,2,3-trimethyl-cyclopent-3-enyl)-hept-5-en-2-one, (*4E*)-7-(2,2,3-trimethyl-cyclopent-3-enyl)-hept-5-en-2-one, (*4Z*)-7-(2,2,3-trimethyl-cyclopent-3-enyl)-hept-5-en-2-one, 2-(6,6-diethoxy-3-ethyl-hexyl)-1,1,5-trimethyl-cyclopentane, 4-(6,6-diethoxy-hex-2-enyl)-1,5,5-trimethyl-cyclopentene, 4-((*2E*)-6,6-diethoxy-hex-2-enyl)-1,5,5-trimethyl-cyclopentene, 4-((*2Z*)-6,6-diethoxy-hex-2-enyl)-1,5,5-trimethyl-cyclopentene.

6. Fragrant or flavouring composition containing at least one campholenic derivative according to any one of claims **1** to **5**, and/or 3-methyl-6-(2,2,3-trimethyl-cyclopent-3-enyl)-hex-4-enal.

7. Use of a campholenic derivative according to anyone of claims **1** to **5**, and/or 3-methyl-6-(2,2,3-trimethyl-cyclopent-3-enyl)-hex-4-enal, or of a composition according to Claim **6**, in perfumery for the preparation of perfumed bases and concentrates, fragrances, and perfumes; topic compositions; cosmetic compositions such as face and body cream, cleansers, facial treatment, talc powders, hair oils, shampoos, hair lotions, bath oils and salts, shower and bath gels, soaps, body anti-perspirants and deodorizers, pre-shave, shaving and post-shave creams and lotions, creams, toothpastes, mouth baths, pomades; cleaning products, such as softeners, detergents, air deodorizers and household cleaning supplies.

8. Use of a campholenic derivative according to anyone of claims **1** to **5**, and/or 3-methyl-6-(2,2,3-trimethyl-cyclopent-3-enyl)-hex-4-enal, or of a composition according to Claim **6**, as flavouring agents for the preparation of flavouring compositions or articles, such as drinks, dairy products, ice creams, soups, sauces, dips, dishes, meat products, culinary assistances, salted biscuits or snacks and also beers, wines and tobaccos.

9. Use of a campholenic derivative according to anyone of claims **1** to **5**, and/or 3-methyl-6-(2,2,3-trimethyl-cyclopent-3-enyl)-hex-4-enal, or of a composition according to Claim **6**, as masking agents of odours and/or flavours, notably in a pharmaceutical, cosmetic or food composition.

10. Use of a campholenic derivative according to anyone of claims **1** to **5**, and/or 3-methyl-6-(2,2,3-trimethyl-cyclopent-3-enyl)-hex-4-enal, or of a composition according to Claim **6**, in combination with other perfumed or flavoured ingredients, solvents or additives.

## Patentansprüche

1. Campholenderivate der allgemeinen Formel (I) 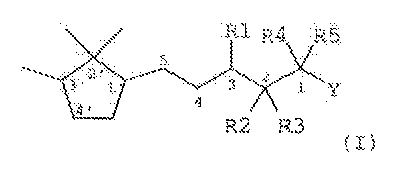 worin
• R1, R2, R3, R4 und R5 jeweils unabhängig für ein Wasserstoffatom oder eine lineare oder verzweigte C₁-C₅-Alkyl- oder C₂-C₅-Alkenylgruppe stehen,
• Y für eine CN-, eine C(O)R₆- oder eine CR₆(OR_{α})(OR_{β})-Gruppe steht,
wobei R₆ für ein Wasserstoffatom oder eine lineare oder verzweigte C₁-C₅-Alkyl- oder C₂-C₅-Alkenylgruppe steht
und R_{α} und R_{β} gleichzeitig für eine lineare oder verzweigte C₁-, C₂-, C₃-, C₄- oder C₅-Alkyl- oder C₂-, C₃-, C₄- oder C₅-Alkenylgruppe stehen und zusammen einen Cyclus bilden können,
• der 5-gliedrige Ring gesättigt ist oder eine Doppelbindung zwischen C3' und C4' in der Formel (I) aufweist und
• die Seitenkette gegebenenfalls eine Doppelbindung zwischen C1 und C2 und/oder zwischen C3 und C4 aufweist,
• mit der Maßgabe, dass das Derivat nicht 3-Methyl-6-(2,2,3-trimethylcyclopent-3-enyl)hex-4-enal ist.

2. Campholenderivate gemäß Anspruch 1 der allgemeinen Formel (la), 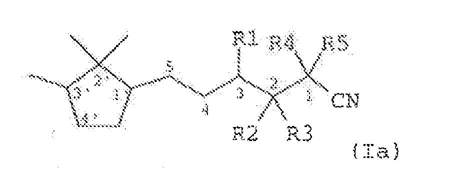 worin
• R1, R2, R3, R4 und R5 jeweils unabhängig für ein Wasserstoffatom oder eine lineare oder verzweigte C₁-C₅-Alkyl- oder C₂-C₅-Alkenylgruppe stehen,
• der 5-gliedrige Ring gesättigt ist oder eine Doppelbindung zwischen C3' und C4' in der Formel (la) aufweist und
• die Seitenkette gegebenenfalls ungesättigt zwischen C1 und C2 und/oder zwischen C3 und C4 in der Formel (la) ist.

3. Campholenderivate gemäß Anspruch **1** der allgemeinen Formel (Ib) 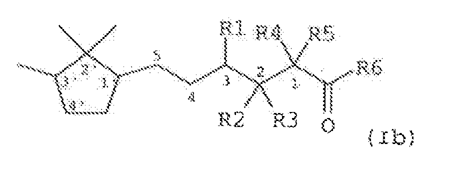 worin
• R1, R2, R3, R4 und R5 jeweils unabhängig für ein Wasserstoffatom oder eine lineare oder verzweigte C₁-C₅-Alkyl- oder C₂-C₅-Alkenylgruppe stehen,
• der 5-gliedrige Ring gesättigt ist oder eine Doppelbindung zwischen C3' und C4' aufweist,
• die Seitenkette gegebenenfalls ungesättigt zwischen C1 und C2 und/oder zwischen C3 und C4 in der Formel (Ib) ist und
• R₆ für ein Wasserstoffatom, eine lineare oder verzweigte C₁-, C₂-, C₃-, C₄- oder C₅-Alkyl- oder C₂-, C₃-, C₄- oder C₅-Alkenylgruppe steht, vorzugsweise R₆ ein Wasserstoffatom oder eine Methylgruppe ist, stärker bevorzugt R₆ ein Wasserstoffatom ist,
• mit der Maßgabe, dass das Derivat nicht 3-Methyl-6-(2,2,3-trimethylcyclopent-3-enyl)hex-4-enal ist.

4. Campholenderivate gemäß Anspruch **1** der allgemeinen Formel (Ic) 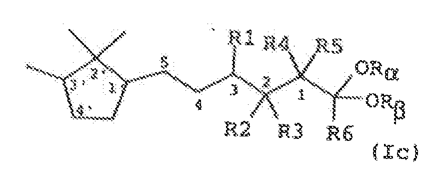 worin
• R1, R2, R3, R4 und R5 jeweils unabhängig für ein Wasserstoffatom oder eine lineare oder verzweigte C₁-C₅-Alkyl- oder C₂-C₅-Alkenylgruppe stehen,
• der 5-gliedrige Ring gesättigt ist oder eine Doppelbindung zwischen C3' und C4' in der Formel (Ic) aufweist,
• die Seitenkette gegebenenfalls ungesättigt zwischen C1 und C2 und/oder zwischen C3 und C4 in der Formel (Ic) ist und
• R_{α} und R_{β} gleichzeitig für eine lineare oder verzweigte C₁-, C₂-, C₃-, C₄- oder C₅-Alkyl- oder C₂-, C₃-, C₄- oder C₅-Alkenylgruppe stehen, vorzugsweise R_{α} und R_{β} gleichzeitig für eine Methyl- oder eine Ethylgruppe stehen,
• R_{α} und R_{β} zusammen einen Cyclus, vorzugsweise einen 5-gliedrigen Ring oder einen 6-gliedrigen Ring, bilden können.

5. Campholenderivate gemäß Anspruch **1**, wobei die Derivate aus der Gruppe gewählt sind, die Folgendes umfasst: 6-(2,2,3-Trimethylcyclopent-enyl)hex-4-ennitril, (*4E*)-6-(2,2,3-Trimethylcyclopent-3-enyl)hex-4-ennitril, (*4Z*)-6-(2,2,3-Trimethylcyclopent-3-enyl)hex-4-ennitril, (+)-6-((1R)-2,2,3-Trimethylcyclopent-3-enyl)hex-4-ennitril, (-)-6-((1S)-2,2,3-Trimethylcyclopent-3-enyl)hex-4-ennitril, (*4E*)-6-((1R)-2,2,3-Trimethylcyclopent-3-enyl)hex-4-ennitril, (*4Z*)-6-((1R)-2,2,3-Trimethylcyclopent-3-enyl)hex-4-ennitril, (*4E*)-6-((1S)-2,2,3-Trimethylcyclopent-3-enyl)hex-4-ennitril, (*4Z*)-6-((1S)-2,2,3-Trimethylcyclopent-3-enyl)hex-4-ennitril, 6-(2,2,3-Trimethylcyclopentyl)hex-4-ennitril, (*4E*)-6-(2,2,3-Trimethylcyclopentyl)hex-4-ennitril, (*4Z*)-6-(2,2,3-Trimethylcyclopentyl)hex-4-ennitril, 4-Methyl-6-(2,2,3-trimethylcyclopent-3-enyl)hex-4-ennitril, (*4Z*)-4-Methyl-6-(2,2,3-trimethylcyclopent-3-enyl)hex-4-ennitril, (*4E*)-4-Methyl-6-(2,2,3-trimethylcyclopent-3-enyl)hex-4-ennitril, 4-Methyl-6-(2,2,3-trimethylcyclopentyl)hexannitril, 4-Ethyl-6-(2,2,3-trimethylcyclopentyl)hexannitril, 4-Methyl-6-(2,2,3-trimethylcyclopentyl)hex-3-ennitril, 6-(2,2,3-Trimethylcyclopent-3-enyl)hex-4-enal, (*4E*)-6-(2,2,3-Trimethylcyclopent-3-enyl)hex-4-enal, (*4Z*)-6-(2,2,3-Trimethylcyclopent-3-enyl)hex-4-enal, (+)-6-((1R)-2,2,3-Trimethylcyclopent-3-enyl)hex-4-enal, (-)-6-((1S)-2,2,3-Trimethylcyclopent-3-enyl)hex-4-enal, (*4Z*)-6-((1R)-2,2,3-Trimethylcyclopent-3-enyl)hex-4-enal, (*4Z*)-6-((1S)-2,2,3-trimethylcyclopent-3-enyl)hex-4-enal, (*4Z*)-6-((1S)-2,2,3-Trimethylcyclopent-3-enyl)hex-4-enal, (*4Z*)-6-((1R)-2,2,3-Trimethylcyclopent-3-enyl)hex-4-enal, 6-(2,2,3-Trimethylcyclopentyl)hex-4-enal, (*4Z*)-6-(2,2,3-Trimethylcyclopentyl)hex-4-enal, (*4E*)-6-(2,2,3-Trimethylcyclopentyl)hex-4-enal, 4-Methyl-6-(2,2,3-trimethylcyclopent-3-enyl)hex-4-enal, (*4E*)-4-Methyl-6-(2,2,3-trimethylcyclopent-3-enyl)hex-4-enal, (*4Z*)-4-Methyl-6-(2,2,3-trimethylcyclopent-3-enyl)hex-4-enal, 4-Methyl-6-(2,2,3-trimethylcyclopentyl)hexanal, 4-Ethyl-6-(2,2,3-trimethylcyclopentyl)hexanal, 3-Methyl-6-(2,2,3-trimethylcyclopentyl)hexanal, 2,4-Dimethyl-6-(2,2,3-trimethylcyclopentyl)hexanal, 7-(2,2,3-Trimethylcyclopent-3-enyl)hept-5-en-2-on, (*4E*)-7-(2,2,3-Trimethylcyclopent-3-enyl)hept-5-en-2-on, (*4Z*)-7-(2,2,3-Trimethylcyclopent-3-enyl)hept-5-en-2-on, 2-(6,6-Diethoxy-3-ethylhexyl)-1,1,5-trimethylcyclopentan, 4-(6,6-Diethoxyhex-2-enyl)-1,5,5-trimethylcyclopenten, 4-((*2E*)-6,6-Diethoxyhex-2-enyl)-1,5,5-trimethylcyclopenten, 4-((2Z)-6,6-Diethoxyhex-2-enyl)-1,5,5-trimethylcyclopenten.

6. Duftstoff- oder Geschmacksstoffzusammensetzung, enthaltend mindestens ein Campholenderivat gemäß einem der Ansprüche **1** bis **5** und/oder 3-Methyl-6-(2,2,3-trimethylcyclopent-3-enyl)hex-4-enal.

7. Verwendung eines Campholenderivats gemäß einem der Ansprüche **1** bis **5** und/oder 3-Methyl-6-(2,2,3-trimethylcyclopent-3-enyl)hex-4-enal oder einer Zusammensetzung gemäß Anspruch **6** in der Parfümerie für die Herstellung von Parfümgrundlagen und -konzentraten, Duftstoffen und Parfümen; topischen Zusammensetzungen; kosmetischen Zusammensetzungen, wie etwa Gesichts- und Körpercreme, Reinigern, Gesichtsbehandlung, Talkpulvern, Haarölen, Shampoos, Haarlotionen, Badeölen und -salzen, Dusch- und Badegelen, Seifen, Körper-Antitranspirantien und Deodorantien, Preshave-, Rasier- und Aftershavecremes und -lotionen, Cremes, Zahnpasten, Mundspülungen, Pomaden; Reinigungsprodukten, wie etwa Weichmachern, Detergentien, Luftdesodorantien und Haushaltsreinigungsbedarf.

8. Verwendung eines Campholenderivats gemäß einem der Ansprüche **1** bis **5** und/oder 3-Methyl-6-(2,2,3-trimethylcyclopent-3-enyl)hex-4-enal oder einer Zusammensetzung gemäß Anspruch **6** als Geschmacksstoffe für die Herstellung von Geschmacksstoffzusammensetzungen oder -artikeln, wie etwa Getränken, Molkereiprodukten, Eiscremes, Suppen, Saucen, Dips, Speisen, Fleischprodukten, kulinarischen Hilfsstoffen, gesalzenen Keksen oder Snacks und auch von Bieren, Weinen und Tabaken.

9. Verwendung eines Campholenderivats gemäß einem der Ansprüche **1** bis **5** und/oder 3-Methyl-6-(2,2,3-trimethylcyclopent-3-enyl)hex-4-enal oder einer Zusammensetzung gemäß Anspruch **6** als Maskierungsmittel von Gerüchen und/oder Aromen, besonders in einer pharmazeutischen, kosmetischen oder Lebensmittelzusammensetzung.

10. Verwendung eines Campholenderivats gemäß einem der Ansprüche **1** bis **5** und/oder 3-Methyl-6-(2,2,3-trimethylcyclopent-3-enyl)hex-4-enal oder einer Zusammensetzung gemäß Anspruch **6** in Kombination mit anderen parfümierten oder aromatisierten Bestandteilen, Lösungsmitteln oder Zusatzstoffen.

## Revendications

1. Les dérivés campholéniques de formule générale (I) 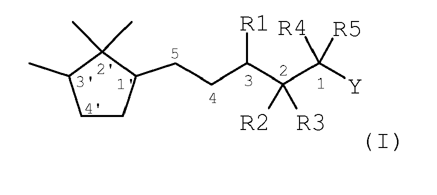 dans laquelle
• R1, R2, R3, R4 et R5 représentent, chacun indépendamment, un atome d'hydrogène ou un groupe, linéaire ou ramifié, alkyle en C1-C5 ou alcényle en C2-C5,
• Y représente un groupe CN, C(O)R6 ou CR6(ORα)(ORβ), où R6 représente un atome d'hydrogène ou un groupe, linéaire ou ramifié, alkyle en C1-C5 ou alcényle en C2-C5,
et Rα et Rβ représentent simultanément un groupe, linéaire ou ramifié, alkyle en C1-C5 ou alcényle en C2-C5 ou forment ensemble un cycle, et de préférence Y représente CN, CHO, C(O)CH3, C(O)C2H5, ou CR6(ORα)(ORβ), où R6 représente un atome d'hydrogène, un méthyle ou un éthyle, et Rα et Rβ représentent simultanément un groupe, linéaire ou ramifié, alkyle en C1-C5 ou alcényle en C2-C5 ou forment ensemble un cycle,
• le cycle en C5 est saturé ou a une double liaison entre C'3 et C'4 dans la formule (I), et
• la chaîne latérale, éventuellement, a une double liaison entre C1 et C2 et / ou entre C3 et C4
• Pourvu que le dérivé n'est pas le 3-méthyl-6-(2,2,3-triméthyl-cyclopent-3-enyl)-hex-4-énal.

2. Les dérivés campholéniques, selon la revendication 1, de formule générale (Ia), 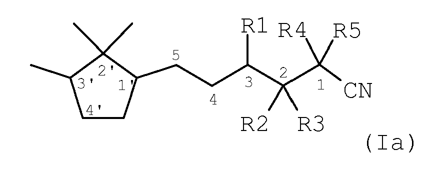 dans laquelle
• R1, R2, R3, R4 et R5 représentent, chacun indépendamment, un atome d'hydrogène ou un groupe, linéaire ou ramifié, alkyle en C1-C5 ou alcényle en C2-C5,
• le cycle en C5 est saturé ou a une double liaison entre C'3 et C'4 dans la formule (Ia), et
• la chaîne latérale est éventuellement insaturée entre C1 et C2 et / ou entre C3 et C4 dans la formule (Ia).

3. Les dérivés campholéniques, selon la revendication 1, de formule générale (Ib), 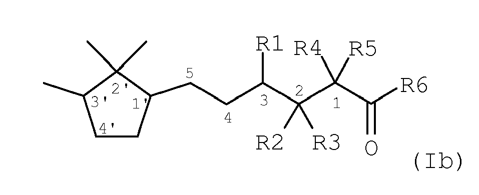 dans laquelle
• R1, R2, R3, R4 et R5 représentent, chacun indépendamment, un atome d'hydrogène ou un groupe, linéaire ou ramifié, alkyle en C1-C5 ou alcényle en C2-C5,
• le cycle en C5 est saturé ou a une double liaison entre C'3 et C'4, et
• la chaîne latérale est éventuellement insaturée entre C1 et C2 et / ou entre C3 et C4 dans la formule (Ib), et
• R6 représente un atome d'hydrogène, un groupe, linéaire ou ramifié, alkyle en C1, C2, C3, C4 ou C5 ou alcényle en C2, C3, C4 ou C5, de préférence, R6 est un atome d'hydrogène ou un groupe méthyle, de préférence R6 est un atome d'hydrogène,
• Pourvu que le dérivé n'est pas 3-méthyl-6-(2,2,3-triméthyl-cyclopent-3-enyl)-hex-4-énal.

4. Les dérivés campholéniques, selon la revendication 1, de formule générale (Ic), 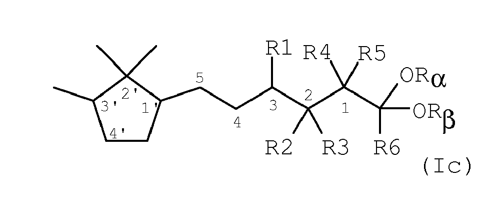 dans laquelle
• R1, R2, R3, R4 et R5 représentent, chacun indépendamment, un atome d'hydrogène ou un groupe, linéaire ou ramifié, alkyle en C1-C5 ou alcényle en C2-C5, de préférence, R1, R2, R3, R4 et R5 représentent, chacun indépendamment, un atome d'hydrogène ou un groupe méthyle ou éthyle,
• le cycle en C5 est saturé ou a une double liaison entre C'3 et C'4 dans la formule (Ic), et
• la chaîne latérale est éventuellement insaturée entre C1 et C2 et / ou entre C3 et C4 dans la formule (Ic), et
• Rα et Rβ représentent simultanément un groupe, linéaire ou ramifié, alkyle en C1-5 ou alcényle en C2-5, de préférence, Rα et Rβ sont simultanément un groupe méthyle ou éthyle, ou
• Rα et Rβ forment ensemble un cycle, de préférence un cycle à 5 ou 6 chaînons

5. Les dérivés campholéniques, selon la revendication 1, **caractérisés en ce que** les-dits dérivés sont sélectionnés dans le groupe comprenant:
6-(2,2,3-triméthyl-cyclopent-3-enyl)-hex-4-ènenitrile, (4E)-6-(2,2,3-triméthyl-cyclopent-3-enyl)-hex-4-ènenitrile, (4Z)-6-(2,2,3-triméthyl-cyclopent-3-enyl)-hex-4-ènenitrile, (+)-6-((1R)-2,2,3-triméthyl-cyclopent-3-enyl)-hex-4-ènenitrile, (-)-6-((1S)-2,2,3-triméthyl-cyclopent-3-enyl)-hex-4-ènenitrile, (4E)-6-((1R)-2,2,3-triméthyl-cyclopent-3-enyl)-hex-4-ènenitrile, (4Z)-6-((1R)-2,2,3-triméthyl-cyclopent-3-ényle )-hex-4-ènenitrile, (4E)-6-((1S)-2,2,3-triméthyl-cyclopent-3-enyl)-hex-4-ènenitrile, (4Z)-6-((1S)-2,2,3-triméthyl-cyclopent-3-enyl)-hex-4-ènenitrile, 6-(2,2,3-triméthyl-cyclopentyl)-hex-4-ènenitrile, (4E)-6-(2 ,2,3-triméthyl-cyclopentyl)-hex-4-ènenitrile, (4Z)-6-(2,2,3-triméthyl-cyclopentyl)-hex-4-ènenitrile, 4-méthyl-6-(2,2 ,3-triméthyl-cyclopent-3-enyl)-hex-4-ènenitrile, (4Z)-4-méthyl-6-(2,2,3-triméthyl-cyclopent-3-enyl)-hex-4-ènenitrile, (4E)-4-méthyl-6-(2,2,3-triméthyl-cyclopent-3-enyl)-hex-4-ènenitrile, 4-méthyl-6-(2,2,3-triméthyl-cyclopentyl)-hexanenitrile, 4-éthyl-6-(2,2,3-triméthyl-cyclopentyl)-hexanenitrile, 4-méthyl-6-(2,2,3-triméthyl-cyclopentyl)-hex-3-ènenitrile, 6 - (2 ,2,3-triméthyl-cyclopent-3-enyl)-hex-4-énal, (4E)-6-(2,2,3-triméthyl-cyclopent-3-enyl)-hex-4-énal, (4Z)-6-(2,2,3-triméthyl-cyclopent-3-enyl)-hex-4-énal, (+)-6-((1R)-2,2,3-triméthyl-cyclopent-3-ényl)-hex-4-énal, (-)-6-((1S)-2,2,3-triméthyl-cyclopent-3-enyl)-hex-4-énal, (4Z)-6-((1R)-2,2,3-triméthyl-cyclopent-3-enyl)-hex-4-énal, (4Z)-6-((1S)-2,2,3-triméthyl-cyclopent-3-enyl)-hex- 4-énal, (4Z)-6-((1S)-2,2,3-triméthyl-cyclopent-3-enyl)-hex-4-énal, (4Z)-6-((1R)-2,2 ,3-triméthyl-cyclopent-3-enyl)-hex-4-énal, 6-(2,2,3-triméthyl-cyclopentyle)-hex-4-énal, (4Z)-6-(2,2,3-triméthyl-cyclopentyl)-hex-4-énal, (4E)-6-(2,2,3-triméthyl-cyclopentyl)-hex-4-énal, 4-méthyl-6-(2,2,3-triméthyl-cyclopent-3-enyl)-hex-4-énal, (4E)-4-méthyl-6-(2,2,3-triméthyl-cyclopent-3-enyl)-hex-4-énal, (4Z)-4-méthyl-6-(2,2,3-triméthyl-cyclopent-3-enyl)-hex-4-énal, 4-méthyl-6-(2,2,3-triméthyl-cyclopentyl)-hexanal, 4-éthyl-6-(2,2,3-triméthyl-cyclopentyl)-hexanal, 3-méthyl-6-(2,2,3-triméthyl-cyclopentyl)-hexanal, 2,4-diméthyl-6-(2,2,3-triméthyl-cyclopentyl)-hexanal, 7-(2,2,3-triméthyl-cyclopent-3-enyl)-hept-5-én-2-one, (4E)-7-(2,2,3-triméthyl-cyclopent-3-enyl)-hept-5-én-2-one, (4Z)-7-(2,2,3-triméthyl-cyclopent-3-enyl)-hept-5-én-2-one, 2-(6,6-diéthoxy-3-éthyl-hexyl)-1,1,5-triméthyl-cyclopentane, 4-(6,6-diéthoxy-hex-2-enyl)-1,5,5-triméthyl-cyclopentène, 4-((2E)-6,6-diéthoxy-hex-2-enyl)-1,5,5-triméthyl-cyclopentène, 4-((2Z)-6,6-diéthoxy-hex-2-ényl)-1,5,5-triméthyl-cyclopentène.

6. Composition parfumante ou aromatisante contenant au moins un dérivé campholénique selon l'une quelconque des revendications 1 à 5, et / ou le 3-méthyl-6-(2,2,3-triméthyl-cyclopent-3-ènyl)-hex-4-ènal.

7. L'utilisation d'un dérivé campholénique selon l'une quelconque des revendications 1 à 5, et / ou le 3-méthyl-6-(2,2,3-triméthyl-cyclopent-3-ènyl)-hex-4-ènal, ou d'une composition selon la revendication 6, en parfumerie pour la préparation de bases parfumées, de concentrés, de parfums, et produits parfumés ; préparations topiques ; préparations cosmétiques telles que crèmes pour le visage et le corps par exemple, nettoyants, soins du visage, poudres de talc, huiles capillaires, shampooings, lotions capillaires, huiles et sels de bain, gels douche et bain, savons, anti-transpirants et déodorants, pré-rasage, et après-rasage, crèmes et lotions, crèmes, dentifrices, bains de bouche, pommades, produits de nettoyage, comme les assouplissants, les détergents, les désodorisants et les produits ménagers.

8. L'utilisation d'un dérivé campholénique selon l'une quelconque des revendications 1 à 5, et / ou le 3-méthyl-6-(2,2,3-triméthyl-cyclopent-3-ènyl)-hex-4-ènal, ou d'une composition selon la revendication 6, comme agent aromatisant pour la préparation de compositions aromatisantes ou articles aromatisés, comme par exemple des boissons, produits laitiers, crèmes glacées, les soupes, les sauces, les trempettes, plats culinaires, produits de viande ou poisson, aides culinaires, biscuits salés ou des collations et des bières, vins et tabacs.

9. L'utilisation d'un dérivé campholénique selon l'une quelconque des revendications 1 à 5, et / ou le 3-méthyl-6-(2,2,3-triméthyl-cyclopent-3-ènyl)-hex-4-ènal, ou d'une composition selon la revendication 6, comme agents masquant d'odeurs et / ou d'arômes, notamment dans les compositions pharmaceutiques, cosmétiques ou alimentaires.

10. L'utilisation d'un dérivé campholénique selon l'une quelconque des revendications 1 à 5, et / ou le 3-méthyl-6-(2,2,3-triméthyl-cyclopent-3-ènyl)-hex-4-ènal, ou d'une composition selon la revendication 6, en association avec tout autre ingrédient parfumant ou aromatisant, solvant ou additif.
